# DEMANDE DE BREVET EUROPEEN

(11) **EP 1 745 773 A1**
(43) Date de publication de la demande: **24.01.2007**
(21) Numéro de dépôt: 06116097.4
(22) Date de dépôt: 26.06.2006
(51) Int. Cl.: A61K 8/81, A61Q 17/04

(54) **Composition photoprotectrice comprenant une phase aqueuse et une cire apolaire de bas point de fusion**

(30) Priorité: 11.07.2005 FR 0552137
(71) Demandeur: L'Oreal, 75008 Paris (FR)
(72) Inventeur: Willemin, Claudie, 75008, Paris (FR); Duffet, Vanessa, 94340, Joinville-le-Pont (FR)
(74) Mandataire: Miszputen, Laurent

(57) **Abrégé**

L'invention concerne une composition comprenant, dans un support cosmétiquement acceptable, au moins un agent filtrant les radiations UV, au moins une phase aqueuse, caractérisée par le fait qu'elle contient au moins une cire apolaire ayant un point de fusion supérieur ou égal à 30°C et une enthalpie de fusion inférieure à 250 J/g (notamment Cirebelle 303, Cirebelle 305, Cirebelle 505).

L'invention concerne également l'utilisation d'au moins une cire apolaire telle que définie dans les revendications précédentes dans une composition comprenant un support cosmétiquement acceptable, au moins une phase aqueuse et au moins un agent filtrant les radiations UV, dans le but de diminuer voire supprimer la brillance et/ou de diminuer voire supprimer l'effet collant et/ou de diminuer voire supprimer le blanchiment et/ou de diminuer voire supprimer le peluchage et/ou d'augmenter le facteur de protection solaire (FPS).

## Description

L'invention concerne une composition comprenant, dans un support cosmétiquement acceptable, au moins un agent filtrant les radiations UV, au moins une phase aqueuse, caractérisée par le fait qu'elle contient au moins une cire apolaire ayant un point de fusion supérieur ou égal à 30°C et une enthalpie de fusion inférieure à 250 J/g.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UV-A et UV-B doivent donc être filtrés et il existe actuellement des compositions cosmétiques protectrices de l'épiderme humain renfermant des filtres UV-A et UVB.

De nombreuses compositions cosmétiques destinées à la photoprotection de la peau ont été proposées. Elles contiennent des filtres UV organiques et de filtres d'origine minérale qui fonctionnent selon leur nature chimique et leurs propriétés physiques, par absorption, réflexion ou diffusion du rayonnement UV. Très souvent, il s'agit de combinaison de filtres organiques liposolubles et /ou hydrosolubles associée à des nanopigments d'oxydes métalliques tels que TiO₂ ou ZnO.

Les compositions antisolaires se présentent assez souvent sous la forme d'une émulsion de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques lipophiles et/ou des filtres organiques classiques hydrophiles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse. D'autre part, les émulsions huile dans eau présentent des qualités sensorielles meilleures que les émulsions eau dans huile, avec un toucher moins gras à l'application sur la peau.

Un des inconvénients majeurs de ces compositions est l'aspect brillant qu'elle confère à la peau après application. Cet aspect est lié à la présence de filtres et à la nature des agents permettant de les solubiliser.

Pour pallier à cet effet de brillance indésirable, on utilise de façon générale des agents matifiants connus à ce jour qui sont soient des charges minérales (telles que SiO₂, ZnO) enrobées ou non, des particules d'amidon (Dry-Flo Plus de National Starch), des billes de polyamide-6 ou 12 (Orgasol R) ou des billes de polyéthylène de haut point de fusion. Et pour être efficaces, ces agents matifiants doivent être introduits à des taux élevés. A ces concentrations élevées ils ont tendance à générer des effets de peluchage et de blanchiment sur la peau.

De plus, l'utilisation de certains agents matifiants comme les cires de polyéthylène rend plus contraignante et plus coûteuse la fabrication des émulsions solaires les contenant dans la mesure où ces cires de haut point de fusion doivent être mises en oeuvre à des températures très supérieures à la température d'émulsification.

Un autre inconvénient à prendre en considération est la difficulté d'atteindre un indice de protection solaire très élevé. Pour cela il s'avère intéressant d'introduire une concentration importante de filtres organiques et/ou minéraux. Mais l'augmentation du taux des filtres organiques présentent le désavantage de laisser un film brillant sur la peau peu cosmétique et peu apprécié par les utilisateurs et peut générer des phénomènes d'intolérance cutanés. Et l'augmentation des filtres minéraux comme les nanopigments d'oxyde métallique (TiO₂) peut produire sur la peau un film résiduel blanc inesthétique. Ce phénomène est lié, le plus souvent, à une mauvaise dispersion des nanopigments dans la formule et une répartition hétérogène sur la peau.

Pour augmenter les indices de protection, on utilise généralement des polymères filmogènes mais le plus souvent ils détériorent les propriétés cosmétiques de la formulation en laissant un film collant, gras et parfois brillant sur la peau ou qui peluche à l'application.

Une autre voie possible est d'introduire des particules minérales mais elles aussi présentent l'inconvénient de conduire aussi à des formulations qui peluchent à l'application.

Une autre voie encore est d'introduire des cires comme les cires en C₁₈-C₃₈ alkylhydroxystéaroylstéarate décrites dans le brevet EP924447, des cires de glycérides citées dans le brevet EP1000611, mais celles-ci confèrent de la brillance aux formules et à l'application.

Il apparaît ainsi nécessaire de disposer de compositions et en particulier des émulsions solaires pouvant être fabriquées dans des conditions douces, qui soient peu brillantes à l'application, ne blanchissent pas, soient non-collantes, ne peluchent pas et assurent une efficacité filtrante optimale.

A la suite d'importantes recherches menées sur la question, il a maintenant été trouvé par la Demanderesse, et ceci de façon tout à fait inattendue et surprenante, qu'il est possible de remédier aux différents inconvénients énumérés ci-dessus, en utilisant au moins un cire apolaire dans un support comprenant au moins une phase aqueuse et un système filtrant les radiations UV. Les compositions antisolaires contenant une telle association présentent en outre une bonne efficacité antisolaire, une bonne rémanence à l'eau, à la transpiration et aux lavages ainsi qu'une bonne persistance dans le temps.

Cette découverte est à la base de la présente invention.

Ainsi, conformément à l'un des objets de la présente invention, il est maintenant proposé une composition comprenant, dans un support cosmétiquement acceptable, au moins un agent filtrant les radiations UV, au moins une phase aqueuse, caractérisée par le fait qu'elle contient au moins une cire apolaire ayant un point de fusion supérieur à 30°C et une enthalpie de fusion inférieure à 250 J/g.

L'invention concerne également l'utilisation d'au moins une cire apolaire ayant un point de fusion supérieur à 30°C et une enthalpie de fusion inférieure à 250 J/g dans une composition comprenant, dans un support cosmétiquement acceptable, au moins une phase aqueuse, dans le but de diminuer voire supprimer la brillance et/ou de diminuer voire supprimer l'effet collant et/ou de diminuer voire supprimer le blanchiment et/ou de diminuer voire supprimer le peluchage et/ou d'augmenter le facteur de protection solaire (FPS).

D'autres caractéristiques, aspects et avantages de l'invention apparaîtront à la lecture de la description détaillée qui va suivre.

Par « agent filtrant les radiations UV », on entend tout composé organique ou minéral unique filtrant les radiations UV ou bien un mélange de plusieurs composés organiques ou minéraux filtrant les radiations UV, par exemple mélange comprenant un filtre UVA et un filtre UVB.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « cire apolaire », on entend toute cire constituée uniquement de molécules ne comportant que des atomes de carbone et d'hydrogène.

Les cires apolaires conformes à l'invention sont généralement des composés lipophiles, solides à température ambiante (25°C), à changement d'état solide/liquide réversible.

Elles présentent un point de fusion supérieur ou égal à 30°C pouvant aller de préférence jusqu'à 80°C et une enthalpie de fusion inférieure à 250J/g. En portant la cire à l'état liquide (fusion), il est possible de la rendre miscible aux huiles et de former un mélange homogène microscopiquement.

En particulier, les cires concernant l'invention présentent un point de fusion supérieur à 40°C et au mieux inférieur ou égal à 70°C.

Plus préférentiellement, leur enthalpie de fusion inférieure ou égale à 240J/g et encore mieux inférieure ou égal à 220 J/g.

Le point de fusion de la cire ainsi que l'enthalpie de fusion peuvent être mesuré à l'aide d'un calorimètre à balayage différentiel (D.S.C) par exemple vendu sous la dénomination MSDSC 2929 par la société TA Instruments. Le point de fusion du composé est la valeur de la température correspondant au pic de la courbe de fusion tandis que l'enthalpie de fusion correspond à l'intégrale de l'ensemble de la courbe de fusion ; la courbe de fusion étant mesurée entre la température de début et celle de la fin de la fusion de ladite cire.

Ne sont pas considérées comme cires apolaires au sens de l'invention, les cires suivantes :
(i) les cires obtenues par estérification ou modifiés par estérification et qui peuvent comprendre des groupes OH résiduels en fonction du rendement de l'estérification. De telles cires sont par exemple issues de la réaction d'un acide gras sur un polyol ramifié de type ditriméthylol comme par exemple celles commercialisé sous la dénomination HEST par la Société HETERENE;
(ii) les cires obtenues de l'hydrogénation catalytiques des huiles animales ou végétales ayant des chaînes grasses, linéaires ou ramifiées en C₈-C₃₂ telles que l'huile de jojoba, l'huile de tournesol hydrogéné; huile de coprah hydrogénée ou la cire obtenue par hydrogénation d'huile d'olive estérifiée avec l'alcool stéarylique ; les cires modifiées siliconées comme la cire de Candellila siliconée commercialisées par KOSTER KEUNEN sous la dénomination Siliconyl candellila ;
(iii) les cires hydrocarbonnées comme la cire d'abeille, la cire de lanoline,la cire d'orange, la cire de citron, la cire de son de riz, la cire de Carnoba, la cire de Candellila, la cire d'Ouricury, la cire du Japon, la cire de Berry, la cire de shellac et la cire de sumac ; la cire de Montan, l'huile ricin hydrogénée ;
(iv) les cires issues de la réaction d'acides gras sur des carbohydrates comme les disaccharides de type sucrose, telles que les polybéhénate de sucrose commercialisée par CRODA sous la dénomination de CROMADERM B ;
(v) les cires hydroxyesters comme par exemple la cire (hydroxystéaryloxy)stéarate d'alkyle en C₂₀-C₄₀ ou SYNTHETIC BEESWAX telles que celles vendues sous les dénominations « KESTER WAX K 82 PR» et « KESTER WAX K80 PR » ;
(vi) les cires de silicone telles que les dimethiconol béhénate et dimethiconol stéarate commercialisées par RHODIA respectivement sous les noms MIRASIL WAX B ET MIRASIL WAX S ;
(vii) les cires apolaires de polyoléfine ayant une enthalpie de fusion supérieure à 250J/g telles que la cire de polyéthylène vendue sous la dénomination PERFORMALENE 400 par NEW "PHASE TECHNOLOGY.

Parmi les cires apolaires utilisables selon l'invention, on peut citer les cires de polyoléfine issues de la polymérisation et notamment d'homopolymérisation d'alpha-oléfine répondant à la formule générale R-CH-CH₂ dans laquelle R désigne un radical alkyle, de préférence, alkyl linéaire, ayant de 10 à 50 atomes de carbones et de préférence de 25 à 50 atomes de carbone.

On entend par homopolymérisation d'alpha-oléfine, la polymérisation de monomères consistant essentiellement en une alpha-oléfine ou un mélange alpha-oléfines. Ces cires ont de préférence un poids moléculaire moyen en nombre allant de 400 à 3000 daltons et particulièrement de 1800 à 2700 daltons.

De telles cires de polyoléfine sont décrites dans les brevets USA-4060569 et US-A-4239546. Ces cires sont notamment vendues sous la dénomination de « PERFORMA V^{R} 103 », « PERFORMA V^{R} 253 », « PERFORMA V^{R} 260 » par la Société NEW PHASE TECHNOLOGIE.

Parmi les cires apolaires utilisables selon l'invention, on peut également citer les cires de paraffine ayant un poids moléculaire moyen en nombre de 350 à 600 daltons comme par exemple le produit commercial vendu sous le nom CERAFINE 56-58 (point de fusion : 56°C et enthalpie de fusion = 232 J/g) par la société BAERLOCHER.

Les cires apolaires conformes à l'invention sont plus particulièrement choisies parmi les cires de polyméthylène susceptibles d'être obtenues par le procédé Fischer-Tropsch.

Elles ont en général un poids moléculaire moyen en nombre allant de 350 à 600 daltons. On utilisera en particulier les cires CIREBELLE fabriquées par la société SASOL comme :
CIREBELLE 303 : point de fusion : 51 °C et enthalpie de fusion = 157-180J/g.
CIREBELLE 305 : point de fusion : 55 °C et enthalpie de fusion = 185J/g.
CIREBELLE 505 : point de fusion : 41 °C et enthalpie de fusion = 177 J/g.

Les cires apolaires conformes à l'invention sont de préférence introduites à un taux compris entre 0,1 et 5% et avantageusement entre 0,5 et 3,0 % et 0,5 et 1,5 % en poids par rapport au poids total de la composition.

Les compositions conformes à l'invention comportent un ou plusieurs agents filtrant les radiations UV choisis parmi les filtres organiques et/ou minéraux actifs dans l'UV-A et/ou l'UV-B hydrophiles et/ou lipophiles et/ou bien insolubles dans les solvants cosmétiques couramment utilisés.

Les filtres UV organiques hydrophiles, lipophiles ou insolubles sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCl :

Dérivés de l'acide para-aminobenzoique :
PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

Dérivés du dibenzoylméthane :
Butyl Methoxydibenzoylmethane vendu notamment sous le nom commercial « PARSOL 1789 » par HOFFMANN LAROCHE,
Isopropyl Dibenzoylmethane,

Dérivés salicyliques:
Homosalate vendu sous le nom « Eusolex HMS » par RONA/EM INDUSTRIES,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par HAARMANN et REIMER,

Dérivés cinnamiques :
Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
- Glyceryl Ethylhexanoate Dimethoxycinnamate

Dérivés de β,β-diphénylacrylate :
Octocrylene vendu notamment sous le nom commercial « UVINUL N539 » par BASF,
Etocrylene, vendu notamment sous le nom commercial « UVINUL N35 » par BASF,

Dérivés de la benzophénone :
Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « HELISORB 11 » par NORQUAY
Benzophenone-8 vendu sous le nom commercial « SPECTRA-SORB UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle vendu sous le nom commercial « UVINUL A+ » par BASF.

Dérivés du benzylidène camphre :
3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :
Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés du phenyl benzotriazole :
Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés de triazine :
Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris-(4'-amino benzalmalonate de diisobutyle)-s- triazine,
2,4,6-tris(4'-amino benzalmalonate de dinéopentyle)-s-triazine
les dérivés de triazine symétriques comme celles décrites notamment dans la demande WO2004/085412, WO2006/034991 en particulier la 2,4,6-tris(biphenyl)-1,3,5-triazine et la la 2,4,6-tris(terphenyl)-1,3,5-triazine.

Dérivés anthraniliques :
Menthyl anthranilate vendu sous le nom commercial commercial « NEO HELIOPAN MA » par Haarmann et REIMER,

Dérivés d'imidazolines :
Ethylhexyl Dimethoxybenzylidene Dioxoimidazoline Propionate,

Dérivés du benzalmalonate :
Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

Dérivés de 4,4-diarylbutadiène :
- 1, 1-dicarboxy (2,2'-diméthyl-propyl)-4,4-diphénylbutadiène

Dérivés de benzoxazole :
2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Les agents filtrant les radiations UV organiques préférentiels sont choisis parmi
Ethylhexyl Methoxycinnamate
Homosalate
Ethylhexyl Salicylate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Camphor Benzalkonium Methosulfate
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
2,4,6-tris-(4'-amino benzalmalonate de diisobutyle)-s-triazine.
2,4,6-tris(biphenyl)-1,3,5-triazine
2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

Les filtres inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des nanopigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « MIRASUN Ti W 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 T, MT 100 TX, MT 100 Z, MT-01 de la société TAYCA, les produits "Solaveil CT-10 W" et "Solaveil CT 100" de la société UNIQEMA et le produit " EUSOLEX T-AVO" de la société MERCK,
- de silice, d'alumine et d'acide alginique tel que le produit "MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO₂ traité par un polydiméthylsiloxane et dont la taille moyenne des particules élémentaires est de 21 nm tel que celui vendu sous la dénomination commerciale "70250 CARDRE UF TIO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-COTE" par la société SUNSMART ;
- ceux commercialisés sous la dénomination "NANOX" par la société Elementis ;
- ceux commercialisés sous la dénomination "NANOGARD WCD 2025" par la société Nanophase Technologies ;

Les pigments d'oxyde de zinc enrobés sont par exemple
- ceux commercialisés sous la dénomination "OXIDE ZINC CS-5" par la société TOSHIBI (ZnO enrobé par polymethylhydrogenesiloxane) ;
- ceux commercialisés sous la dénomination "NANOGARD ZINC OXIDE FN" par la société Nanophase Technologies (en dispersion à 40% dans le Finsolv TN, benzoate d'alcools en C₁₂-C₁₅);
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société DAIKIN (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société SHIN-ETSU (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHODIA.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les agents filtrant les radiations UV sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « ELDEW SL-205 » par la société AJINOMOTO), les acides ou les esters gras (comme le benzoate d'alcools en C₁₂-C₁₅ vendu sous la dénomination commerciale « FINSOLV TN » ou « WITCONOL TN » par la société WITCO, le palmitate d'octyle, le lanolate d'isopropyle, les triglycérides dont ceux des acides caprique/caprylique, le dicaprylyl carbonate vendu sous la dénomination « Cetiol CC » par la société COGNIS), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C₁₀-C₃₀-alkylacrylate); les polyacrylamides comme par exemple les copolymères réticulés vendus sous les noms SEPIGEL 305 (nom C.T.F.A. : polyacrylamide/C13-14 isoparaffin/Laureth 7) ou SIMULGEL 600 (nom C.T.F.A. : acrylamide / sodium acryloyldimethyltaurate copolymer /isohexadecane / polysorbate 80) par la société SEPPIC ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société HOECHST sous la dénomination commerciale « HOSTACERIN AMPS » (nom CTFA : ammonium polyacryldimethyltauramide) ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que le poly C₁₀-C₃₀ alkyl acrylate vendu sous la dénomination « DORESCO IPA 13-1 » par la société LANDEC ou encore les argiles modifiées telles que le l'hectorite et ses dérivés, comme les produits commercialisés sous les noms de Bentone.

Parmi les actifs, on peut citer :
- les vitamines (A, C, E, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile ou bien d'une dispersion.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société DOW CORNING, et les alkyl-dimethicone copolyols tels que le Laurylmethicone copolyol vendu sous la dénomination "DOW CORNING 5200 Formulation Aid" par la société DOW CORNING ; le Cetyl dimethicone copolyol tel que le produit vendu sous la dénomination Abil EM 90R par la société GOLDSCHMIDT et le mélange de cétyl diméthicone copolyol, d'isostearate de polyglycérole (4 moles) et de laurate d'hexyle vendu sous la dénomination ABIL WE 09 par la société GOLDSCHMIDT. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom ARLACEL P135 par la socité ICI ;

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination ARLACEL 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives PLANTAREN 2000 et PLANTAREN 1200, le cétostéarylglucoside éventuellement en mélange avec l'alcool cétostéarylique, commercialisé par exemple sous la dénomination MONTANOV 68 par la société SEPPIC, sous la dénomination TEGOCARE CG90 par la société Goldschmidt et sous la dénomination EMULGADE KE3302 par la société Henkel, ainsi que l'arachidyl glucoside, par exemple sous la forme du mélange d'alcools arachidique et béhénique et d'arachidylglucoside commercialisé sous la dénomination MONTANOV 202 par la société SEPPIC. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de soin et/ou de protection solaire pour le visage et/ou le corps de consistance liquide à semi-liquide, telles que des lotions, des laits, des crèmes plus ou moins onctueuses, des gels, des gel-crèmes. Elles peuvent éventuellement être conditionnées en aérosol et se présenter sous forme de mousse ou de spray.

Les compositions cosmétiques selon l'invention peuvent par exemple être utilisées comme produit de maquillage.

Les compositions selon l'invention sous forme de lotions fluides vaporisables conformes à l'invention sont appliquées sur la peau ou les cheveux sous forme de fines particules au moyen de dispositifs de pressurisation.

Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés. L'invention va maintenant être décrite en référence aux exemples suivants donnés à titre illustratif et non limitatif.

Dans ces exemples, sauf indication contraire, les quantités sont exprimées en pourcentages pondéraux.

On a réalisé les formulations solaires suivantes ; les quantités sont indiquées en pourcentages en poids :

### Exemples 1 à 3 : Emulsions H/E

| **Composition** | **Ex 1 (*) %pds** | **Ex 2 %pds** | **Ex 3 (*) %pds** |
|---|---|---|---|
| **Phase huileuse : A** | | | |
| MELANGE MONO/DISTEARATE DE GLYCERYLE / STEARATE DE POLYETHYLENE GLYCOL (100 OE) | 1 | 1 | 1 |
| ACIDE STEARIQUE | 1 | 1 | 1 |
| ISO-HEXADECANE | 2 | 2 | 2 |
| BENZOATE D'ALCOOLS C12/C15 | 3 | 3 | 3 |
| CYCLOPENTASILOXANE | 5 | 5 | 5 |
| OCTOCRYLENE | 3 | 3 | 3 |
| BUTYL METHOXYDIBENZOYLMETHANE | 0,5 | 0,5 | 0,5 |
| TiO₂ | 1 | 1 | 1 |
| COPOLYMERE ACIDE ACRYLIQUE/METHACRYLATE DE STEARYLE POLYMERISE DANS UN MELANGE ACETATE D'ETHYLE/CYCLOHEXANE | 0,25 | 0,25 | 0,25 |
| XANTHANE | 0,1 | 0,1 | 0,1 |
| COPOLYMERE VINYLPYRROLIDONE / EICOSENE | 1 | 1 | 1 |
| POLYDIMETHYLSILOXANE | 0,5 | 0,5 | 0,5 |
| VITAMINE E | 0.1 | 0,1 | 0.1 |
| CIRE DE POLYMÉTHYLÈNE (Cirebelle 303) | 0 | 1 | 0 |
| SYNTHETIC BEES WAX (Kesterwax K82P) | 0 | 0 | 1 |

| **Phase aqueuse :B** | | | |
|---|---|---|---|
| TEREPHTHALYLIDENE DICAMPHOR SULFONIC ACID | 0,9 | 0,9 | 0,9 |
| GLYCEROL | 6 | 6 | 6 |
| PROPYLENE GLYCOL | 6 | 6 | 6 |
| TRIETHANOLAMINE | 0,71 | 0,71 | 0,71 |
| PHOSPHATE DE MONO-CETYLE MONO-POTASSIQUE | 1 | 1 | 1 |
| EAU | qsp 100 | qsp 100 | qsp 100 |

| **Phase C :** | | | |
|---|---|---|---|
| CONSERVATEUR | 1,25 | 1,25 | 1,25 |
| CHELATANT | 0.1 | 0.1 | 0.1 |
| PARFUM | | | 1 |

| | | | |
|---|---|---|---|
| (*) hors invention : - l'exemple 1 ne contient pas de cire - l'exemple 3 contient la cire SYNTHETIC BEES WAX qui est une cire polaire du type cire (hydroxystéaryloxy)stéarate d'alkyle en C₂₀-C₄₀ | | | |

### Mode de préparation

On chauffe séparément les phases A et B à 60-70°C, on réalise l'émulsion sous agitation type rotor-stator et on introduit ensuite la phase C. On laisse revenir l'émulsion à température ambiante sous agitation modérée.

### Mesure de matité

La mesure de réflexion est réalisée à l'aide d'un gonioréflectomètre sur un film d'épaisseur de 30 µm préalablement séché pendant 60 minutes à température ambiante.

La composition est étalée sur une carte de contraste (Prufkarte type 24/5-250cm² commercialisée par la société Ericksen) à l'aide d'un tire-film.

Le résultat obtenu est le rapport R entre la réflexion spéculaire et la réflexion diffuse. La valeur de R est d'autant plus faible que l'effet matifiant est important.

| **Composition** | **Ex 1** | **Ex 2** | **Ex 3** |
|---|---|---|---|
| R | 28,15 ± 1.16 | 7,93 ± 0.1 | 34,28 ± 0.1 |

A la concentration de 1% c'est la Cirebelle 303 (composition 2) permet de réduire la brillance de la formule témoin, tandis que la SYNTHETIC BEES WAX ou KESTER WAX K82P augmente la brillance.

### Efficacité filtrante

Le facteur de protection solaire (FPS) pour les compositions 1, 2 et 3 été déterminé selon le protocole Colipa 2003

Les valeurs de FPS obtenues sont les suivantes :

| **formule** | **Ex 1** | **Ex 2** | **Ex 3** |
|---|---|---|---|
| FPS in vivo | 5,9 ± 0.9 | 13,1 ± 2.5 | 10,7 ± 1,7 |

Les résultats de ces tests montrent que la composition 2 selon l'invention contenant la cire apolaire de polyméthylène de point de fusion supérieur 51 °C et ayant une enthalpie de fusion égale à 157-180J/g permet à la fois de diminuer sensiblement la brillance et d'augmenter le facteur de protection solaire par rapport à la composition 1 ne contenant pas de cire contrairement à la composition 3 contenant une cire polaire.

### Exemples 5 : Lait anti-solaire E/H

| **Composition** | **Ex5 Invention** |
|---|---|
| **Phase huileuse A₁** | |
| PEG-30 DIPOLYHYDROXYSTEARATE | 2 |
| C12-15 ALKYL BENZOATE | 7,5 |
| ISOHEXADECANE | 5 |
| OCTOCRYLENE | 2,5 |
| ETHYLHEXYL SALICYLATE | 5 |
| BIS-ETHYLHEXYLOXYPHENOL METHOXYPHENYL TRIAZINE | 1 |
| BUTYL METHOXYDIBENZOYLMETHANE | 3,5 |
| ETHYLHEXYL TRIAZONE | 1 |
| SYHNTHETIC WAX : Cirebelle 303 | 1 |
| TiO₂ | 5 |

| **Phase huileuse A₂** | |
|---|---|
| LAURYL PEG/PPG-18/18 METHICONE | 1,82 |
| ISOSTEARYL ALCOHOL | 0,18 |
| CYCLOHEXASILOXANE | 6 |
| CYCLOPENTASILOXANE | 6 |
| ETHYLHEXYLGLYCERIN | 0,5 |
| VITAMINE E | 0,2 |

| **Phase aqueuse** | |
|---|---|
| TEREPHTHALYLIDENE DICAMPHOR SULFONIC ACID | 1,5 |
| TRIETHANOLAMINE | 0,78 |
| GLYCERIN | 2 |
| PROPYLENE GLYCOL | 4 |
| CHELATANT | 0,3 |
| ALCOHOL | 6 |
| EAU | qsp |

### Mode de préparation

On chauffe au bain-marie à 85°C, la phase A₁ (excepté TiO₂) et à 65°C la phase aqueuse sous agitation. On disperse le TiO₂ dans la phase A₁ sous agitation rotor-stator. On Introduit la phase A₂ dans A₁. On réalise l'émulsion à 65-70°C en introduisant la phase aqueuse sous forte agitation rotor-stator. On laisse refroidir à température ambiante avant d'ajouter l'alcool.

La composition ainsi obtenue est mate à l'application, ne blanchit pas, est non collante, ne peluche pas et produit une efficacité filtrante satisfaisante.

## Revendications

1. Composition comprenant, dans un support cosmétiquement acceptable, au moins un agent filtrant les radiations UV, au moins une phase aqueuse, **caractérisée par le fait qu'**elle contient au moins une cire apolaire ayant un point de fusion supérieur ou égal à 30°C et une enthalpie de fusion inférieure à 250 J/g.

2. Composition selon la revendication 1, où la cire apolaire a un point de fusion allant de 30 à 80°C.

3. Composition selon la revendication 2, où la cire apolaire présente un point de fusion supérieur à 40°C et au mieux inférieur ou égal à 70°C.

4. Composition selon l'une quelconque des revendications 1 à 3, où la cire apolaire a une enthalpie de fusion inférieure ou égale à 240J/g.

5. Composition selon la revendication 4, où la cire apolaire a une enthalpie de fusion inférieure ou égale à 220J/g.

6. Composition selon l'une quelconque des revendications précédentes, où la cire apolaire est choisie parmi les cires de polyoléfine issues de la polymérisation et notamment d'homopolymérisation d'alpha-oléfine répondant à la formule générale R-CH-CH₂ dans laquelle R désigne un radical alkyle ayant de 10 à 50 atomes de carbones.

7. Composition selon la revendication 4, où la cire de polyoléfine présente un poids moléculaire moyen en nombre allant de 400 à 3000 daltons et particulièrement de 1800 à 2700 daltons.

8. Composition selon l'une quelconque des revendications précédentes, où la cire apolaire est choisie parmi les cires de paraffine ayant un poids moléculaire moyen en nombre de 350 à 600 daltons.

9. Composition selon l'une quelconque des revendications précédentes, où la cire apolaire est choisie parmi les cires de polyméthylène susceptibles d'être obtenues par le procédé Fischer-Tropsch.

10. Composition selon la revendication 4, où la cire de polyméthylène présente un poids moléculaire moyen en nombre allant de allant de 350 à 600 daltons.

11. Composition selon l'une quelconque des revendications précédentes, où la ou les cires apolaires sont présentes à un taux allant de 0,1 à 5% en poids et avantageusement de 0,5 à 3% en poids et plus particulièrement de 0,5 à 1,5% en poids par rapport au poids total de la composition.

12. Composition selon l'une quelconque des revendications précédentes, où l'agent filtrant les radiations UV est choisis parmi les filtres UV organiques et/ou minéraux actifs dans l'UVA et/ou l'UVB hydrophiles et/ou lipophiles et/ou bien insolubles dans les solvants cosmétiques couramment utilisés.

13. Composition selon la revendication 12, où les filtres UV organiques sont choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène; les 4,4-diarylbutadiènes et leurs mélanges.

14. Composition selon la revendication 13, où les filtres UV organiques sont choisis parmi choisis parmi
Ethylhexyl Methoxycinnamate
Homosalate
Ethylhexyl Salicylate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Camphor Benzalkonium Methosulfate
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine.
2,4,6-tris(biphenyl)-1,3,5-triazine
2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

15. Composition selon la revendication 12, où les filtres UV inorganiques sont des pigments ou des nanopigments d'oxydes métalliques, traités ou non.

16. Composition selon la revendication 15, **caractérisée par le fait que** lesdits pigments ou nanopigments sont choisis parmi les oxydes de titane, de zinc, de fer, de zirconium, de cérium et leurs mélanges, traités ou non.

17. Composition selon l'une quelconque des revendications 1 à 16, **caractérisée par le fait qu'**elle comprend en outre au moins un agent de bronzage et/ou de brunissage artificiel de la peau.

18. Composition selon l'une quelconque des revendications 1 à 17, **caractérisée par le fait qu'**elle comprend en outre un ou plusieurs adjuvants cosmétiques choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants.

19. Composition selon l'une quelconque des revendications 1 à 18, **caractérisée par le fait qu'**elle se présente sous la forme d'une émulsion huile-dans-eau ou eau-dans huile ou d'une dispersion.

20. Utilisation d'une composition telle que définie dans l'une quelconque des revendications 1 à 19 pour la fabrication de produits pour le traitement cosmétique de la peau, des lèvres, des ongles, des cheveux, des cils, sourcils et/ou du cuir chevelu.

21. Utilisation d'une composition telle que définie dans l'une quelconque des revendications 1 à 19 pour la fabrication de produits de soin de la peau, des lèvres, des ongles, des cheveux et/ou du cuir chevelu.

22. Utilisation d'une composition telle que définie dans l'une quelconque des revendications 1 à 19 pour la fabrication de produits de protection solaire des lèvres, des ongles, des cheveux et/ou du cuir chevelu.

23. Utilisation d'une composition telle que définie dans l'une quelconque des revendications 1 à 19 pour la fabrication de produits de maquillage.

24. Utilisation d'au moins une cire apolaire telle que définie dans les revendications précédentes dans une composition comprenant un support cosmétiquement acceptable, au moins une phase aqueuse et au moins un agent filtrant les radiations UV, dans le but de diminuer voire supprimer la brillance et/ou de diminuer voire supprimer l'effet collant et/ou de diminuer voire supprimer le blanchiment et/ou de diminuer voire supprimer le peluchage et/ou d'augmenter le facteur de protection solaire (FPS).
